# EUROPÄISCHE PATENTSCHRIFT

(11) **EP 1 962 735 B1**
(45) Veröffentlichungstag und Bekanntmachung des Hinweises auf die Patenterteilung: **14.09.2011**
(21) Anmeldenummer: 06828672.3
(22) Anmeldetag: 14.12.2006
(51) Int. Cl.: A61F 2/66

(54) **KÜNSTLICHER FUSS**
ARTIFICIAL FOOT
PIED ARTIFICIEL

(30) Priorität: 22.12.2005 DE 102005062231
(43) Veröffentlichungstag der Anmeldung: 03.09.2008
(73) Patentinhaber: Otto Bock HealthCare GmbH, 37115 Duderstadt (DE)
(72) Erfinder: MOSLER, Lüder, 37115 Duderstadt (DE)
(74) Vertreter: Lins, Edgar
(86) Internationale Anmeldenummer: PCT/DE2006/002241
(87) Internationale Veröffentlichungsnummer: WO 2007/076807

(56) Entgegenhaltungen:
- DE-A1- 10 010 302
- FR-A- 486 030
- GB-A- 2 311 466
- US-A- 2 529 968
- US-A- 2 749 557

## Beschreibung

Die Erfindung betrifft einen künstlichen Fuß mit einem Anschlussteil für ein Unterschenkelteil, einem Vorfußteil, das mittels eines oberen Koppelelements und eines unteren Koppelelements mit dem Anschlussteil in einer Viergelenk-Anordnung derart gelenkig verbunden ist, dass eine Winkelstellung des Anschlussteils eine Winkelstellung des Vorfußteils steuert, wobei sich eines der Koppelelemente bis in einen Fersenbereich des Fußes hinein erstreckt.

Ein derartiger künstlicher Fuß ist in der nicht vorveröffentlichten deutschen Patentanmeldung gemäß DE 10 2004 031 562 A1 (WO-A-2006/000211, ein Dokument das unter Art. 54(3) EPÜ fällt) beschrieben, auf deren Offenbarung Bezug genommen wird. Durch die Kopplung des Vorfußteils mit dem Anschlussteil für das Unterschenkelteil über die beiden Koppelelemente erfolgt erstmalig eine Zwangskopplung zwischen dem Anschlussteil und dem Vorfußteil bezüglich der Winkelstellung. Eine Veränderung der Winkelstellung des Anschlussteils - bzw. des Unterschenkeltells - nach posterior führt zu einem proportionalen Anheben des Vorfußteils. Daher kann der künstliche Fuß von einer Stellung ohne Absatz ("Barfuß-Stellung") in einen Schuh mit einem hohen Absatz eingeführt werden, wodurch sich die Stellung des Unterschenkels - und damit des Anschlussteils - relativ zur Sohlenfläche des künstlichen Fußes im Sinne einer Verstellung des Winkels nach posterior verändert. Dadurch wird das Vorfußteil soweit angehoben, dass es wieder parallel zur Auflagefläche (vordere Sohle des Schuhs) liegt. Das Vorfußteil liegt daher fest auf der Standfläche auf und nimmt die benötigte Last für ein sicheres Stehen, auch mit dem hohen Absatz auf. Eine früher vorgesehene weiche oder elastische Ausbildung des Vorfußteils ist daher nicht erforderlich.

Der vorbeschriebene künstliche Fuß enthält vorzugsweise eine Mehrgelenk-Anordnung, insbesondere eine Viergelenk-Anordnung, wobei die Koppelelemente zwischen dem Vorfuß und einem Mittelfußbereich im Wesentlichen parallel zueinander liegen können. Hierdurch ergibt sich eine 1:1 Kopplung der Winkelbewegungen von Anschlussteil und Vorfußteil, die für eine Absatzhöhenanpassung ideal ist. Bei dem vorbeschriebenen künstlichen Fuß erstreckt sich das obere Koppelelement in den Fersenbereich hinein und ist somit als eine Art Wippe ausgebildet, die etwa mittig gelenkig am Anschlussteil gelagert ist. Diese Konstruktion hat den Vorteil, dass das Drehgelenk des oberen Koppelgliedes etwa in der Position des natürlichen Knöchelgelenks angeordnet ist. Nachteilig ist allerdings, dass die Belastungsbewegung des als Wippe ausgebildeten oberen Koppelgliedes die Fußlänge des künstlichen Fußes im Sohlenbereich verändert, sodass beispielsweise bei einer wippenden Belastung ein ungewolltes Wandern des Fußes auftreten kann.

Durch US 2,529,968 ist eine Prothese bekannt, bei der ein künstlicher Fuß aus einem Hauptfußteil und einem gelenkig daran angebrachten Vorfußteil ausgebildet ist. Das Hauptfußteil ist mit einem Unterschenkelteil Ober ein Drehgelenk verbunden, das eine Bewegung des Unterschenkelteils relativ zum Hauptfußteil aus einer gestreckten Standstellung nur nach posterior erlaubt. Diese Bewegung wird bedämpft durch eine am Unterschenkelteil angebrachte Druckfeder, die mit einem vorderen Teil des Hauptfußteils über ein Kabel verbunden ist. Bei einer Gehbewegung behält der Unterschenkel beim Abrollen nach vorn seine rechtwinkelige Stellung zu dem Hauptfußteil bei und hebt mit seinem Fersenbereich vom Boden ab. Das gelenkig am Hauptfußteil befestigte Vorfußteil liegt auf dem Boden auf, verändert also seinen Winkel relativ zum Haupt-Fußteil. Diese Bewegung wird bedämpft durch ein weiteres Kabel, das mit einer weiteren Druckfeder auf der posterioren Seite des Unterschenkelteils verbunden ist. Dadurch wird eine Abrollbewegung des Hauptfußteils bewirkt, bei der das Vorfußteil auf dem Boden unter einem zunehmenden Druck der Druckfeder liegt und dadurch für eine Stabilisierung der Gehbewegung beim Abrollen über die Zehen des Fußes gewährleistet. Eine komplette Kopplung des Winkels des Vorfußteils zum Hauptfußteil mit dem Winkel zwischen dem Unterschenkelteil und dem Hauptfußteil ist dabei weder vorgesehen noch möglich. Es fehlt ferner an einem Anschlussteil für das Unterschenkelteil, das mit dem Hauptfußteil gelenkig verbunden ist.

DE 100 10 302 A1 offenbart einen Prothesenfuß, bei dem sich ein Hauptfußteil in den Fersenbereich hinein erstreckt und an seinem vorderen Ende ein Drehgelenk für eine gelenkige Verbindung mit einem Vorfußteil aufweist. Ferner ist im Bereich des Knöchelgelenks ein Drehgelenk vorgesehen, mit dem ein Anschlussteil für ein Unterschenkelteil gelenkig befestigbar ist. Um beim Abrollen über den Vorfuß beim Gehzyklus ein starkes Absenken des Hüftgelenks zu vermeiden, ist zwischen dem Vorfußteil und dem Hauptfußteil eine Schubstange angeordnet, die sich bei einer Veränderung des Winkels des Vorfußteils gegenüber dem Hauptfußteil dadurch bewegen kann, dass ein Langloch der Schubstange ein am Hauptfußteil fixiertes Lager führt. An einer Lagerfläche an einer oberen Längsseite der Schubstange kann ein Lager anliegen, das auf dem Anschlussteil fixiert ist. Beim Gehen rollt sich der Fuß durch Anheben des Fersenteils ab, wobei der Vorfuß auf der Lauffläche anliegt. Die dadurch bewirkte Winkelverstellung zwischen Vorfußteil und Hauptfußteil schiebt die Schubstange in Richtung Hauptfußteil, wodurch beim Abrollen über den Vorfuß der Ausgangswinkel von 90° zwischen dem Hauptfußteil und dem Anschlussteil nach posterior verringert wird, sodass eine zu starke Absenkung des Hüftgelenks vermieden wird. Die Schubstange bewirkt somit eine Veränderung des Winkels zwischen dem starr mit dem Unterschenkelteil verbindbaren Anschlussteil und dem Hauptfußteil. In einer Variante wirkt die Schubstange mit ihrem über das Langloch hinausragenden Ende mit einem weitern Lenker zusammen, der einerseits mit dem Ende der Schubstange und andererseits mit dem Anschlussteil gelenkig verbunden ist. Eine Kopplung zwischen dem Vorfußteil und dem Anschlussteil ist dabei nicht direkt möglich und vorgesehen, da es wesentlich auf die Verbindung des Vorfußteils mit dem Hauptfußteil durch die Schubstange ankommt. Durch den Zwischenlenker entsteht eine Fünfgelenk-Anordnung. Für eine in der Druckschrift nicht vorgesehene Steuerung des Winkels des Vorfußteils in Abhängigkeit von dem Winkel des Anschlussteils relativ zum Hauptfußteil (zum unteren Koppelelement) ist die bekannte Konstruktion nicht geeignet.

Durch FR 486 030 ist ein von einem Hauptfußteil abklappbares Vorfußteil bekannt, dass bei einer Belastung des Vorfußes beim Abrollen mit Hilfe einer Lenker-Zahnradanordnung eine Verriegelung des Winkels des Vorfußteils relativ zum Hauptfußteil bewirkt. Diese Verriegelung wird erst durch das Aufsetzen des Fußes auf die Ferse beim nächsten Gehzyklus aufgehoben. Eine proportionale Kopplung des Winkels des Vorfußteils zum Winkel des Unterschenkelteils ist daher nicht vorgesehen.

Der vorliegenden Erfindung liegt daher die Aufgabe zugrunde, die Vorteile eines künstliches Fußes der eingangs erwähnten Art zu erzielen, ohne die Nachteile einer Fußlängenveränderung durch die Bewegung des oberen Koppelgliedes in Kauf nehmen zu müssen.

Zur Lösung dieser Aufgabe ist erfindungsgemäß ein künstlicher Fuß der eingangs erwähnten Art dadurch gekennzeichnet, dass sich das untere der Koppelelemente bis in den Fersenbereich hinein erstreckt. Bei dem erfindungsgemäßen künstlichen Fuß verursacht die Bewegung des Vorfußteils keine Längenänderung des Fußes, sodass der durch die Fußlängenveränderung entstehende Nachteil vermieden wird. Da für die Knöchelbewegung des erfindungsgemäßen künstlichen Fußes das Gelenk des unteren Koppelgliedes maßgeblich ist, kann der erfindungsgemäße Vorteil dazu führen, dass das Gelenk des unteren Koppelgliedes etwas unter der Lage des physiologischen Knöchelgelenks liegt, weil aus kosmetischen Gründen ein wesentlich höherer Aufbau der Fußkonstruktion nicht erwünscht ist. Bezüglich der Lage des Drehgelenks muss in diesem Fall eine Kompromisslösung zugrunde gelegt werden.

Der erfindungsgemäße künstliche Fuß erlaubt eine automatische Anpassung der Winkelstellung des Vorfußteils zu der Auflagefläche der Sohle des künstlichen Fußes im Übrigen, die durch die Auflagepunkte "Ballen" am Verbindungspunkt zwischen Vorfußteil und den den Mittelfußbereich bildenden Koppelelementen einerseits und "Ferse" am hinteren Ende des Fersenbereichs andererseits definiert ist. In einem Schuh mit einem erhöhten Absatz steht diese Auflagefläche schräg zur Horizontalen. Da sich das Unterschenkelteil zum Erhalt des Gleichgewichts im Wesentlichen lotrecht ausrichten muss, entsteht dabei ein nach posterior verdrehter Winkel zwischen dem unteren Koppelelement und dem Anschlussteil für das Unterschenkelteil. Dieser Winkel wird auf das Vorfußteil übertragen und sorgt für eine Anhebung des Vorfußteils, sodass dieses flächig parallel zum Boden bzw. zum vorderen Sohlenbereich eines Schuhs ausgerichtet ist und im Vorfußbereich dann Kräfte aufnehmen kann, die eine hohe Standsicherheit vermitteln.

Wie bei der vorbeschriebenen Konstruktion gemäß DE 10 2004 031 562 A1 kann es zweckmäßig sein, eines der Koppelelemente in Grenzen längenveränderlich auszubilden. Für das Gehen ist eine direkte Übertragung der Winkelbewegung des Knöchels auf das Vorfußteil nicht vorteilhaft. Vorzuziehen ist eine durch eine Schrägstellung der Koppelelemente erfolgende Übersetzung, die die Winkelbewegungen des Knöchels überproportional in Bewegungen des Vorfußteils übersetzt. In diesem Fall ist für den Ausgleich der Absatzhöhe eine Längenanpassung eines der Koppelglieder erforderlich. Diese kann statisch erfolgen, sodass eine Längenanpassung nur bei einem Schuhwechsel zur Anpassung an die Absatzhöhe erfolgt. Es ist jedoch auch eine dynamische Längenänderung möglich, indem eins der Koppelelemente elastisch längbar oder beispielsweise durch einen Hydraulikzylinder längenverstellbar ist.

Dadurch ist es beispielsweise möglich, die bei der Vorverlagerung des Gewichts auftretende starke Vorfußbelastung zu mindern. Ist die Längenveränderung gegen die Wirkung einer zunehmenden elastischen Gegenkraft möglich, entsteht eine progressiv anwachsende Vorfuß-Gegenkraft, wie sie gewünscht ist. Ein gewisses "Schwanken" beim Stehen des Patienten wird dabei mit einer elastischen, progressiv anwachsenden Gegenkraft stabilisiert, was einem natürlichen Stehgefühl entspricht.

Die Längenveränderung des Koppelelements kann auch durch einen Hydraulikzylinder bewirkt werden, vorzugsweise durch einen Hydraulikzylinder, der nur durch langsame Änderungen verstellbar ist und bei kurzzeitigen Belastungen blockiert. Ein derartiger Hydraulikzylinder ist in der DE 10 2004 031 562 A1 ausführlich erläutert.

Für die Gangdynamik ist es vorteilhaft, wenn die relative Bewegung zwischen dem Fersenbereich und dem Anschlusselement durch eine Dämpferanordnung bedämpft ist. Die Dämpferanordnung soll dabei eine dämpfende Federfunktion aufweisen, kann vorzugsweise aber auch so ausgebildet sein, dass sie auf statische Belastungen reagiert, indem ein Hydraulikzylinder mit einer Umströmungsöffnung vorgesehen ist. Auf diese Weise kann die Anpassung an unterschiedliche Absatzhöhen so erfolgen, dass die jeweilige Standposition kräftefrei ist und nicht gegen eine etwaige Gegenkraft gehalten werden muss.

Es kann zweckmäßig sein, die gelenkige Befestigung der Koppelelemente nicht durch Drehgelenke zu bewirken, sondern durch eine federelastische Anbindung. Dabei kann die federelastische Anbindung nur an einem Ende des Koppelelements, aber auch an beiden Enden des Koppelelements ausgebildet sein.

In einer besonders bevorzugten Ausführungsform der Erfindung liegt das untere Koppelelement federelastisch an dem Vorfußteil und an dem Anschlussteil mit jeweils einem Blattfederelement an, wobei die beiden Blattfederelemente in den Fersenbereich geführt sind und dort flächig aneinander anliegen. Dabei bildet das mit dem Anschlusselement verbundene Blattfederelement eine C-Form aus, während das mit dem Vorfußteil verbundene Blattfederelement eine leichte Wellenform aufweist.

Der erfindungsgemäße künstliche Fuß eignet sich ferner für eine zusätzliche dynamische Einstellung, bei der ein Koppelelement in seinen Dämpfungseigenschaften kontinuierlich, beispielsweise bei dem Einsatz eines Hydraulikzylinders mit einem elektronisch verstellbaren Ventil, steuerbar ist. Die Steuerung kann dabei in Abhängigkeit von gemessenen Belastungs- und Lagezuständen des Fußes erfolgen.

Die vorliegende Erfindung soll im Folgenden anhand von in der Zeichnung dargestellten Ausführungsbeispielen näher erläutert werden. Es zeigen:
- Figur 1: eine Seitenansicht eines ersten Ausführungsbeispiels eines erfindungsgemäßen künstlichen Fußes;
- Figur 2: eine Seitenansicht der modifizierten ersten Ausführungsform;
- Figur 3: eine zweite Ausführungsform des erfindungsgemäßen künst- lichen Fußes;
- Figur 4: eine dritte Ausführungsform des erfindungsgemäßen künstli- chen Fußes.

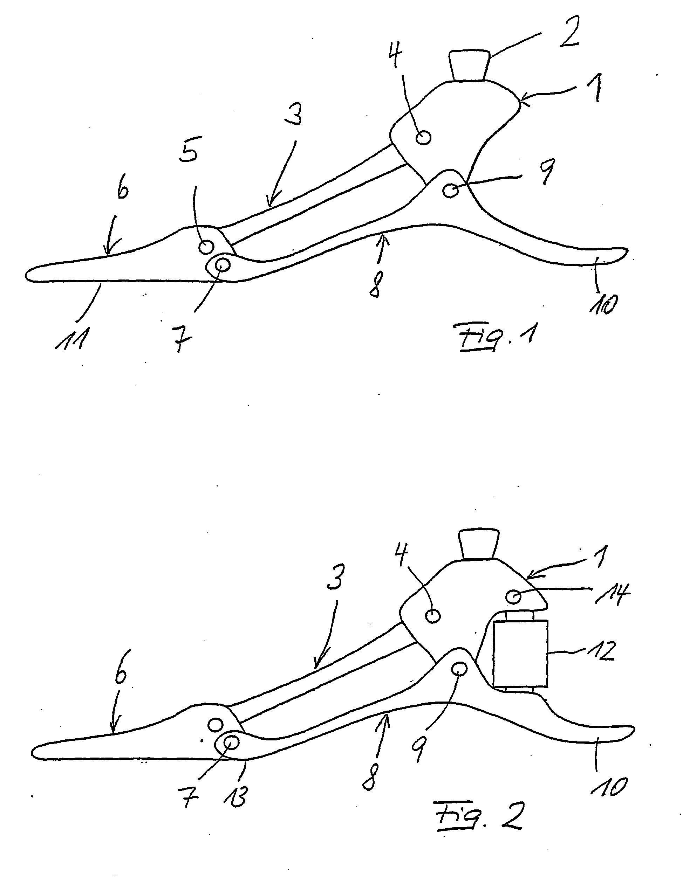

Die in Figur 1 dargestellte erste Ausführungsform eines erfindungsgemäßen Fußes weist ein Anschlussteil 1 auf, das an seiner Oberseite mit einem Adapteransatz 2 in Form eines auf dem Kopf stehenden Kegelstumpfes versehen ist. Der Adapteransatz 2 ermöglicht eine justierbare Aufnahme eines rohrförmigen Unterschenkelteils einer Unterschenkelprothese.

Mit dem Anschlussteil 1 ist ein oberes Koppelelement 3 durch ein Drehgelenk 4 verbunden. Das andere Ende des oberen Koppelelements ist über ein weiteres Drehgelenk 5 mit einem Vorfußteil 6 verbunden, mit dem der Zehenbereich eines natürlichen Fußes nachgeahmt wird. An dem Vorfußteil 6 befindet sich unterhalb des Drehgelenks 5 ein weiteres Drehgelenk 7, über das ein unteres Koppelelement 8 mit dem Vorfußteil 6 verbunden ist. Das untere Koppelelement 8 ist über ein Drehgelenk 9 an ein unteres Ende des Anschlussteils 1 angelenkt und weist einen das untere Koppelelement 8 über das Drehgelenk 9 hinaus in ein freies Fersenende 10 fortsetzenden Ansatz auf, der in einen Fersenbereich des Fußes ragt. Das freie Fersenende 10 ist daher etwas nach unten abgewinkelt. Es ist erkennbar, dass das untere Koppelelement 8 mit seinem hinteren freien Fersenende 10 und den beide Drehgelenken 7, 9 eine Wippe bildet, die beispielsweise beim Aufsetzung des Fußes auf die Ferse zur Durchführung eines Schrittes durch die Bodenwiderstandskraft am freien Fersenende 10 belastet wird und somit ein Drehmoment auf das Vorfußteil 6 ausübt, durch das das Vorfußteil 6 nach oben verschwenkt wird. Das Drehgelenk 9 der Wippe entspricht dem Knöchelgelenk.

Das Vorfußteil 6 bildet einen etwa dreieckförmigen Keil aus, dessen Unterseite 11 parallel zu einer (nicht dargestellten) Auflagefläche für den Fuß im Bereich des Vorfußteils 6 bildet.

In dem in Figur 1 dargestellten Ausführungsbeispiel sind das Anschlussteil 1, die Koppelelemente 3, 8 und das Vorfußteil 6 starr ausgebildet. Eine Belastung im Bereich des Fersenendes 10 führt somit zu einem sofortigen Hochdrehen des Vorfußteils 6, das einem Anheben der Zehen eines Fußes entspricht. Um diese unnatürliche Reaktion zu mildern, ist in dem modifizierten Ausführungsbeispiel gemäß Figur 2 ein Dämpfer 12 vorgesehen, der zwischen dem Anschlussteil 1 und dem Fersenende 10 des unteren Koppelelements 8 angeordnet ist. Bei einer Beaufschlagung des Fersenendes 10 mit einer Bodenreaktionskraft sorgt der Dämpfer 12 beispielsweise für eine verzögerte und ggf. begrenzte Verschwenkung des hinteren Fersenendes 10 nach oben, wodurch eine entsprechend verzögerte und begrenzte Verschwenkung des Vorfußteils 6 nach oben bewirkt wird.

Der Dämpfer 12 ist vorzugsweise als ein Hydraulikzylinder mit einer Umströmungsöffnung vorgesehen, die durch eine zu hohe Strömungsgeschwindigkeit verschlossen wird. Dadurch ist es möglich, die Winkelstellung zwischen dem Ansatzteil 1 und dem hinteren Fersenende 10 bzw. dem unteren Koppelelement 8 quasi statisch zu ändern, wenn beispielsweise der künstliche Fuß in einen Schuh mit einem erhöhten Absatz eingebracht wird. Auf diese Weise ist es möglich, die Winkelstellung des Anschlussteils 1, und damit des Unterschenkelteils einer Prothese, relativ zu dem unteren Koppelelement 8 anzupassen, um bei einem aufgrund eines erhöhten Absatzes weg stehenden unteren Koppelelements 8 eine lotrechte Ausrichtung des Unterschenkelteils zu ermöglichen, die nicht gegen elastische Reaktionskräfte gehalten werden muss.

Die Winkelstellung des unteren Koppelelements 8 ergibt sich im Übrigen aus den Auflageflächen, die sich am hinteren Fersenende 10 einerseits und an dem mit dem Drehgelenk 7 versehenen vorderen Ballenende 13 ergeben.

Zu erwähnen ist ferner, dass der Dämpfer 12 ebenfalls gelenkig mit dem hinteren Fersenende 10 und dem Anschlussteil 1 verbunden ist. Figur 2 lässt ein entsprechendes Drehgelenk 14 im Anschlussteil 1 erkennen.

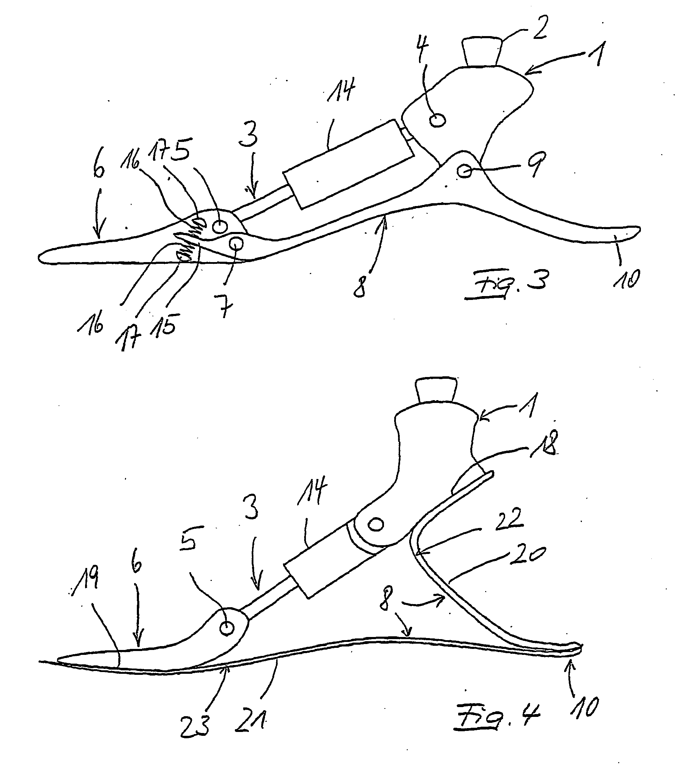

Bei dem in Figur 3 dargestellten Ausführungsbeispiel ist das obere Koppelelement 3' mittels eines Hydraulikzylinders 14 längenveränderlich ausgebildet. Diese Ausführungsform trägt zur Anpassung des künstlichen Fußes an unterschiedliche Absatzhöhen eines Schuhs bei. Ferner ist das untere Koppelelement 8 mit einem Ansatz 15 versehen, der das untere Koppelelement 8 über das Drehgelenk 7 hinaus in ein freies Ende fortsetzt und beidseitig jeweils eine Druckfeder 16 beaufschlagt, die mit einem stationären Anschlag 17 zusammenwirkt. Die Verschwenkung des unteren Koppelelements 8 relativ zu dem Vorfußteil 6 wird durch die Druckfedern 16 elastisch bedämpft und durch die Anschläge 17 begrenzt, um eine übermäßige Verschwenkung des Vorfußteils 6 zu vermeiden.

Bei der in Figur 4 dargestellten dritten Ausführungsform eines erfindungsgemäßen Fußes sind das Anschlussteil 1 und das Vorfußteil 6 jeweils mit einer Anlagefläche 18, 19 versehen, an der ein entsprechender Abschnitt jeweils eines Blattfederelements 20, 21 anliegt und dort entsprechend befestigt ist. Die beiden Blattfederelemente 20, 21 bilden zusammen das untere Koppelelement 8, das somit elastisch ausgebildet ist. Das mit dem Anschlussteil 1 verbundene Blattfederelement 20 ist etwa C-förmig ausgebildet und verwirklicht gleichzeitig ein federndes Fersenelement, übt also eine dem Dämpfer 12 in Figur 2 ähnliche Funktion aus. Das mit dem Vorfußteil 6 verbundene Blattfederelement 21 erstreckt sich nach Art einer Sohle wellenförmig vom Vorfußteil 6 zum Fersenende 10, an dem beide Blattfederelemente 20, 21 flächig aneinander anliegen, miteinander verbunden sind und ein freies Ende gemeinsam bilden.

Aufgrund der Formgebung der Blattfederelemente 20, 21 entstehen Drehpunkte 22, 23. Der Drehpunkt 22 liegt nahe am Anschlussteil 1 und übt die Knöchelgelenkfunktion aus, während der Drehpunkt 23 auf dem Blattfederelement 21 im Bereich des Ballens des künstlichen Fußes liegt.

Es ist erkennbar, dass bei den in den Figuren 1 bis 4 dargestellten Ausführungsformen eine Zwangssteuerung des Schwenkwinkels des Vorfußteils 6 bewirkt wird, wobei jedoch die wirksame Fußlänge unverändert bleibt.

## Patentansprüche

1. Künstlicher Fuß mit einem Anschlussteil (1) für ein Unterschenkelteil, einem Vorfußteil (6), das mittels eines oberen Koppelelements (3) und eines unteren Koppelelements (8) mit dem Anschlussteil (1) in einer Viergelenk-Anordnung (4, 5, 7, 9) derart gelenkig verbunden ist, dass eine Winkelstellung des Anschlussteils (1) eine Winkelstellung des Vorfußteils (6) steuert, wobei sich eines der Koppelelemente (3, 8) bis in einen Fersenbereich des Fußes hinein erstreckt, wobei sich das untere Koppelement (8) bis in den Fersenbereich hinein erstreckt.

2. Künstlicher Fuß nach Anspruch 1, wobei eines der Koppelelemente (3, 8) längenveränderlich ausgebildet ist.

3. Künstlicher Fuß nach Anspruch 2, wobei das längenveränderliche Koppelelement (3, 8) gegen eine zunehmende elastische Gegenkraft längbar ausgebildet ist.

4. Künstlicher Fuß nach Anspruch 2, wobei das längenveränderliche Koppelelement (3) einen Hydraulikzylinder (14) aufweist.

5. Künstlicher Fuß nach einem der Ansprüche 2 bis 4, wobei das obere Koppelelement (3) längenveränderlich ausgebildet ist.

6. Künstlicher Fuß nach einem der Ansprüche 1 bis 5, wobei eine Dämpferanordnung (12) zwischen dem sich in den Fersenbereich erstreckenden Koppelement (8) und dem Anschlussteil (1) angeordnet ist.

7. Künstlicher Fuß nach einem der Ansprüche 1 bis 6, wobei die gelenkige Verbindung eines der Koppelelemente (8) durch wenigstens eine federelastische Anbindung ausgebildet ist.

8. Künstlicher Fuß nach Anspruch 7, wobei das Koppelelement (8) sowohl an dem Vorfußteil (6) als auch an dem Anschlussteil (1) federelastisch anliegt.

9. Künstlicher Fuß nach Anspruch 8, wobei das untere Koppelelement (8) federelastisch an dem Vorfußteil (6) und an dem Anschlussteil (1) mit jeweils einem Blattfederelement (20, 21) anliegt und dass die Blattfederelemente (20, 21) in den Fersenbereich geführt sind und dort flächig aneinander anliegen.

10. Künstlicher Fuß nach einem der Ansprüche 1 bis 9, wobei die Bewegung des Vorfußteils (6) gegenüber dem unteren Koppelglied (8) durch eine zwischengeschaltete federelastische Anschlageinrichtung (16, 17) begrenzt ist.

11. Künstlicher Fuß nach einem der Ansprüche 1 bis 10, wobei die Dämpfungseigenschaften eines Koppelelements (3, 8) in Abhängigkeit von gemessenen Belastungs- und/oder Lagezuständen des Fußes einstellbar ausgebildet sind.

## Claims

1. Artificial foot with a connecting part (1) for a lower leg part, a forefoot part (6) which is pivotably connected to the connecting part (1) in a four-joint arrangement (4, 5, 7, 9) by means of an upper coupling element (3) and a lower coupling element (8) in such a way that an angular position of the connecting part (1) controls an angular position of the forefoot part (6), one of the coupling elements (3, 8) extending into a heel area of the foot, wherein the lower coupling element (8) extends into the heel area.

2. Artificial foot according to Claim 1, wherein one of the coupling elements (3, 8) is designed to be adjustable in length.

3. Artificial foot according to Claim 2, wherein the length-adjustable coupling element (3, 8) is designed to be extendable against an increasing elastic counter-force.

4. Artificial foot according to Claim 2, wherein the length-adjustable coupling element (3) has a hydraulic cylinder (14).

5. Artificial foot according to one of Claims 2 to 4, wherein the upper coupling element (3) is designed to be adjustable in length.

6. Artificial foot according to one of Claims 1 to 5, wherein a damper arrangement (12) is arranged between the connecting part (1) and the coupling element (8) extending into the heel area.

7. Artificial foot according to one of Claims 1 to 6, wherein the articulated connection of one of the coupling elements (8) is formed by at least one resilient link.

8. Artificial foot according to Claim 7, wherein the coupling element (8) bears resiliently both on the forefoot part (6) and also on the connecting part (1).

9. Artificial foot according to Claim 8, wherein the lower coupling element (8) bears resiliently on the forefoot part (6) and on the connecting part (1) in each case via a leaf spring element (20, 21), and in that the leaf spring elements (20, 21) are guided into the heel area where their surfaces bear on each other.

10. Artificial foot according to one of Claims 1 to 9, wherein the movement of the forefoot part (6) relative to the lower coupling part (8) is limited by an interposed resilient stop device (16, 17).

11. Artificial foot according to one of Claims 1 to 10, wherein the damping properties of a coupling element (3, 8) are designed to be adjustable as a function of measured loading states and/or positional states of the foot.

## Revendications

1. Pied artificiel comprenant une partie de raccordement (1) pour une partie de bas de la jambe, une partie avant-pied (6) qui est reliée articulée, au moyen d'un élément de couplage supérieur (3) et d'un élément de couplage inférieur (8), à la partie de raccordement (1) dans un agencement à quatre barres (4, 5, 7, 9) de sorte qu'une position angulaire de la partie de raccordement (1) pilote une position angulaire de la partie avant-pied (6), l'un des éléments de couplage (3, 8) s'étendant jusque dans une région du talon du pied, l'élément de couplage inférieur (8) s'étendant jusque dans la région du talon.

2. Pied artificiel selon la revendication 1, dans lequel l'un des éléments de couplage (3, 8) est formé modifiable en longueur.

3. Pied artificiel selon la revendication 2, dans lequel l'élément de couplage (3, 8) modifiable en longueur est formé allongeable contre une force élastique opposée croissante.

4. Pied artificiel selon la revendication 2, dans lequel l'élément de couplage (3) modifiable en longueur présente un cylindre hydraulique (14).

5. Pied artificiel selon l'une quelconque des revendications 2 à 4, dans lequel l'élément de couplage supérieur (3) est formé modifiable en longueur.

6. Pied artificiel selon l'une quelconque des revendications 1 à 5, dans lequel un agencement d'amortissement (12) est agencé entre l'élément de couplage (8) s'étendant jusque dans la région du talon, et la partie de raccordement (1).

7. Pied artificiel selon l'une quelconque des revendications 1 à 6, dans lequel la liaison articulée de l'un des éléments de couplage (8) est formée par au moins une attache élastique.

8. Pied artificiel selon la revendication 7, dans lequel l'élément de couplage (8) s'appuie élastiquement sur la partie avant-pied (6) comme sur la partie de raccordement (1).

9. Pied artificiel selon la revendication 8, dans lequel l'élément de couplage inférieur (8) s'appuie élastiquement sur la partie avant-pied (6) et sur la partie de raccordement (1) avec un élément ressort à lame (20, 21) respectif, et les éléments ressort à lame (20, 21) vont dans la région du talon et s'y appuient à plat l'un sur l'autre.

10. Pied artificiel selon l'une quelconque des revendications 1 à 9, dans lequel le mouvement de la partie avant-pied (6) est délimité relativement à l'organe de couplage inférieur (8) par un dispositif de butée (16, 17) élastique intercalé.

11. Pied artificiel selon l'une quelconque des revendications 1 à 10, dans lequel les propriétés d'amortissement d'un élément de couplage (3, 8) sont réglables en fonction de conditions de charge et/ou de position mesurées du pied.
